# EUROPEAN PATENT APPLICATION

(11) **EP 3 444 597 A1**
(43) Date of publication of application: **20.02.2019**
(21) Application number: 17781959.6
(22) Date of filing: 17.04.2017
(51) Int. Cl.: G01N 27/12

(54) **ALARM CIRCUIT OF GAS-SENSITIVE SENSOR**

(30) Priority: 15.04.2016 CN 201610237530
(71) Applicant: Institute of Process Engineering, Chinese Academy of Sciences, Beijing 100190 (CN)
(72) Inventor: HAN, Ning, Beijing 100190 (CN); WU, Xiaofeng, Beijing 100190 (CN); CHEN, Yunfa, Beijing 100190 (CN)
(74) Representative: Prinz & Partner mbB
(86) International application number: PCT/CN2017/080805
(87) International publication number: WO 2017/177983

(57) **Abstract**

A self-feedback circuit includes a gas-sensitive sensor and a voltage dividing circuit. The voltage dividing circuit includes a field effect transistor. A gas-sensitive resistor of the gas-sensitive sensor is connected in series to a source electrode and a drain electrode of the field effect transistor. An increased one between a divided voltage across the gas-sensitive resistor and a divided voltage across the voltage dividing circuit is used as a output voltage of the self-feedback circuit.

## Description

### TECHNICAL FIELD

An alarm circuit with a gas-sensitive sensor is provided, which belongs to the field of electronics such as a gas-sensitive sensor and an alarm.

### BACKGROUND

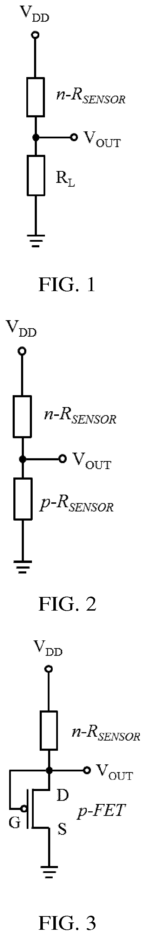

The semiconductor gas-sensitive sensor plays an important role in the detection and alarm of flammable and explosive gases, toxic and harmful gases, polluted gases, and gases concerning food hygiene. The operating principle of the semiconductor gas-sensitive sensor is that resistance of the semiconductor material changes with the concentration of the gas to be detected at a certain temperature. As shown in FIG. 1, V_{DD} is a test voltage, V_{OUT} is an output voltage signal, RL is a load resistor, and n-R_{SENSOR} is an n-type oxide semiconductor gas-sensitive sensor. The change of the gas-sensitive sensor resistor n-R_{SENSOR} will cause the change of current in the circuit. The n-R_{SENSOR} is connected in series to the load resistor RL, which eventually causes the change of divided voltage across the load resistor RL or the gas-sensitive sensor n-R_{SENSOR}. The change of divided voltage may drive an external circuit to give an alarm or display concentration information of the gas to be detected via the digital-to-analog conversion. In the related art, commercial alarms (such as Zhengzhou Winsen MP503 and Japanese Figaro TGS2602) and most research papers and patents (such as CN103729970A and CN204129920U) use this kind of simple and easy to operate series circuit. In addition, apart from the load resistor with a fixed resistance, part products also use a resistor with a manually adjustable resistance (such as CN103558260B), so as to flexibly adjust the circuit parameter.

In addition, in some academic papers or books, the load resistor may also be replaced by a gas-sensitive sensor. As shown in FIG 2, V_{DD} is a test voltage, V_{OUT} is an output voltage signal, p-R_{SENSOR} is a p-type oxide semiconductor gas-sensitive sensor, and n-R_{SENSOR} is an n-type oxide semiconductor gas-sensitive sensor. The sensitivity and specific responsiveness for detecting gases may be improved by matching characteristic parameters of two gas-sensitive sensors n-R_{SENSOR} and p-R_{SENSOR} (Han et al., Sensors and Actuators B: Chemical, 2009, 138:228; Wu Xinghui and Wang Caijun, Sensor and Signal Processing, Publishing House of Electronics Industry, 1997).

### SUMMARY

An alarm circuit with a gas-sensitive sensor provided by the present disclosure can improve the intelligence of the alarm circuit with the gas-sensitive sensor and promote the alarm capability of the circuit via self-feedback effect of a field effect transistor. The self-feedback effect of the field effect transistor is a difference between the alarm circuit in the present disclosure and the alarm circuit in the related art.

The present disclosure provides a self-feedback circuit including a gas-sensitive sensor and a voltage dividing circuit. The voltage dividing circuit includes a field effect transistor. A source electrode and a drain electrode of the field effect transistor are connected in series to each other. One of a divided voltage across a gas-sensitive resistor and a divided voltage across the voltage dividing circuit, which is increased, is used as a output voltage of the self-feedback circuit.

Optionally, a gate electrode of the field effect transistor is shorted to the source electrode or the drain electrode of the field effect transistor; and one of the divided voltage across the gas-sensitive resistor and a divided voltage across the field effect transistor, which is increased, is used as the output voltage.

Optionally, when a p-channel field effect transistor is applied, the gate electrode of the p-channel field effect transistor is shorted to the drain electrode of the p-channel field effect transistor; and when an n-channel field effect transistor is applied, the gate electrode of the n-channel field effect transistor is shorted to the source electrode of the n-channel field effect transistor.

Optionally, when an n-type semiconductor gas-sensitive sensor is applied to test a reducing gas and a p-type semiconductor gas-sensitive sensor is applied to test an oxidizing gas, the divided voltage across the field effect transistor is used as the output voltage; and when the n-type semiconductor gas-sensitive sensor is applied to test the oxidizing gas and the p-type semiconductor gas-sensitive sensor is applied to test the reducing gas, the divided voltage across the gas-sensitive sensor is used as the output voltage.

Optionally, the field effect transistor is an enhancement mode field effect transistor or a depletion mode field effect transistor.

Optionally, the gas-sensitive sensor is a sensor which operates at room temperature, or a gas-sensitive sensor which operates at a certain temperature after being heated by a heating circuit.

Optionally, the voltage dividing circuit includes the field effect transistor and a series resistor, the gas-sensitive resistor of the gas-sensitive sensor is connected in series to the source electrode and the drain electrode of the field effect transistor via the series resistor; the source electrode or the drain electrode of the field effect transistor is connected in series to a gate electrode of the field effect transistor via the series resistor; and one of the divided voltage across the gas-sensitive resistor and a common divided voltage across the field effect transistor and the series resistor, which is increased, is used as the output voltage.

Optionally, when an n-type semiconductor gas-sensitive sensor is applied to test a reducing gas and a p-type semiconductor gas-sensitive sensor is applied to test an oxidizing gas, the common divided voltage across the field effect transistor and the series resistor is used as the output voltage; and when the n-type semiconductor gas-sensitive sensor is applied to test the oxidizing gas and the p-type semiconductor gas-sensitive sensor is applied to test the reducing gas, the divided voltage across the gas-sensitive sensor is used as the output voltage.

Optionally, when the field effect transistor is a p-channel field effect transistor, the drain electrode of the p-channel field effect transistor is connected to the gate electrode of the p-channel field effect transistor via the series resistor; and when the field effect transistor is an n-channel field effect transistor, the source electrode of the n-channel field effect transistor is connected to the gate electrode of the n-channel field effect transistor via the series resistor.

Optionally, when an n-type semiconductor gas-sensitive sensor is applied to test a reducing gas and a p-type semiconductor gas-sensitive sensor is applied to test an oxidizing gas, the common divided voltage across the field effect transistor and the series resistor is used as the output voltage; and when the n-type semiconductor gas-sensitive sensor is applied to test the oxidizing gas and the p-type semiconductor gas-sensitive sensor is applied to test the reducing gas, the divided voltage across the gas-sensitive sensor is used as the output voltage.

Optionally, the field effect transistor is an enhancement mode field effect transistor or a depletion mode field effect transistor.

Optionally, the gas-sensitive sensor is a sensor which operates at room temperature, or a gas-sensitive sensor which operates at a certain temperature after being heated by a heating circuit.

Optionally, a gate electrode of the field effect transistor is shorted to the source electrode or the drain electrode of the field effect transistor; and one of a the divided voltage across the gas-sensitive resistor and a divided voltage across the field effect transistor, which is decreased, is used as the output voltage.

Optionally, the voltage dividing circuit includes the field effect transistor and a series resistor, the gas-sensitive resistor of the gas-sensitive sensor is connected in series to the source electrode and the drain electrode of the field effect transistor via the series resistor; the source electrode or the drain electrode of the field effect transistor is connected to a gate electrode of the field effect transistor via the series resistor; and one of the divided voltage across the gas-sensitive resistor and a common divided voltage across the field effect transistor and the series resistor, which is decreased, is used as the output voltage.

Optionally, when the field effect transistor is a p-channel field effect transistor, the gate electrode of the p-channel field effect transistor is shorted to the drain electrode of the p-channel field effect transistor; and when the field effect transistor is an n-channel field effect transistor, the gate electrode of the n-channel field effect transistor is shorted to the source electrode of the n-channel field effect transistor.

Optionally, when an n-type semiconductor gas-sensitive sensor is applied to test a reducing gas and a p-type semiconductor gas-sensitive sensor is applied to test an oxidizing gas, the divided voltage across the gas-sensitive sensor is used as the output voltage; and when the n-type semiconductor gas-sensitive sensor is applied to test the oxidizing gas and the p-type semiconductor gas-sensitive sensor is applied to test the reducing gas, the divided voltage across the field effect transistor is used as the output voltage.

Optionally, when the field effect transistor is a p-channel field effect transistor, the gate electrode of the p-channel field effect transistor is shorted to the drain electrode of the p-channel field effect transistor; and when the field effect transistor is an n-channel field effect transistor, the gate electrode of the n-channel field effect transistor is shorted to the source electrode of the n-channel field effect transistor.

Optionally, when an n-type semiconductor gas-sensitive sensor is applied to test a reducing gas and a p-type semiconductor gas-sensitive sensor is applied to test an oxidizing gas, the divided voltage across the gas-sensitive sensor is used as the output voltage; and when the n-type semiconductor gas-sensitive sensor is applied to test the oxidizing gas and the p-type semiconductor gas-sensitive sensor is applied to test the reducing gas, the divided voltage across the field effect transistor is used as the output voltage.

The present disclosure further provides an alarm circuit with a gas-sensitive sensor, including the self-feedback circuit described above and an alarm display circuit. The alarm display circuit includes at least one of a buzzer, a light-emitting diode, LED, and a relay, an operational amplifier and a triode, and the output voltage of the self-feedback circuit is connected to the operational amplifier and the triode to drive the at least one of the buzzer, the LED and the relay to give an alarm; or the alarm display circuit includes a voltage reader, the output voltage is connected to the voltage reader, the output voltage is used as an alarm voltage, and the voltage reader is configured to digitalize the alarm voltage and output the digitalized alarm voltage. The operating principle of the alarm circuit with the gas-sensitive sensor connected in the method described above is that: when the gas-sensitive sensor is detecting a gas to be detected, the resistance of the gas-sensitive resistor of the gas-sensitive sensor changes. In the case where the direct current test voltage is constant, change of resistance causes change of current in the circuit, causing the change of divided voltage across series devices. Because the gate electrode of the field effect transistor is shorted to the source electrode or the drain electrode, or the gate electrode of the field effect transistor is connected to the source electrode or the drain electrode via the series resistor, change of divided voltage across the field effect transistor causes change of gate voltage. The gate voltage exerts a self-feedback effect on the change of the current in the circuit, therefore enhancing the change of the divided voltage caused by the change of resistance of the gas-sensitive sensor. With continuous self-feedback adjustment, the output voltage of the alarm voltage in the steady state is greater than the output voltage of the load voltage circuit using a fixed resistor in the related art, thereby improving alarm capability of the circuit.

### BRIEF DESCRIPTION OF DRAWINGS

Embodiments will be described below in detail with reference to the accompanying drawings 1 to 13.
FIG. 1 is a self-feedback circuit using an n-type gas-sensitive sensor with a fixed resistor to detect a reducing gas in the related art;
FIG. 2 is a self-feedback circuit using a p-type gas-sensitive sensor and an n-type gas-sensitive sensor to detect a reducing gas in the related art;
FIG. 3 is a self-feedback circuit using an n-type gas-sensitive sensor and a p-type field effect transistor connected in series to detect a reducing gas according to an embodiment 1;
FIG. 4 is a self-feedback circuit using a p-type gas-sensitive sensor and a p-type field effect transistor connected in series to detect a reducing gas according to an embodiment 2 ;
FIG. 5 is a self-feedback circuit using a p-type gas-sensitive sensor and an n-type field effect transistor connected in series to detect a reducing gas according to an embodiment 3;
FIG. 6 is a self-feedback circuit using an n-type gas-sensitive sensor and an n-type field effect transistor connected in series to detect a reducing gas according to an embodiment 4;
FIG. 7 is a self-feedback circuit using a p-type gas-sensitive sensor and a p-type field effect transistor connected in series to detect an oxidizing gas according to an embodiment 5;
FIG. 8 is a self-feedback circuit using an n-type gas-sensitive sensor and a p-type field effect transistor connected in series to detect an oxidizing gas according to an embodiment 6;
FIG. 9 is a self-feedback circuit using a p-type gas-sensitive sensor and an n-type field effect transistor connected in series to detect an oxidizing gas according to an embodiment 7;
FIG. 10 is a self-feedback circuit using an n-type gas-sensitive sensor and an n-type field effect transistor connected in series to detect an oxidizing gas according to an embodiment 8;
FIG. 11 an alarm circuit 1 of a gas-sensitive sensor according to an embodiment;
FIG. 12 an alarm circuit 2 of a gas-sensitive sensor according to an embodiment; and
FIG. 13 an alarm circuit 3 of a gas-sensitive sensor according to an embodiment.

### DETAILED DESCRIPTION

If not in collision, the following embodiments and the features thereof may be combined with each other.

The present disclosure will be described in detail in combination with embodiments. In order to make the circuit clear, a heating circuit which is included in a sensor resistor is not shown.

### Embodiment 1

As shown in FIG. 3, V_{DD} is a test voltage, V_{OUT} is an output voltage signal, n-R_{SENSOR} is an n-type oxide semiconductor gas-sensitive resistor, p-FET is a p-type field effect transistor, and G, D and S are respectively a gate electrode, a drain electrode and a source electrode. The gas-sensitive resistor n-R_{SENSOR} of the n-type oxide semiconductor SnO₂ gas-sensitive sensor is connected in series to the p-type field effect transistor p-FET, a first end of the gas-sensitive resistor is connected to a positive electrode of the test voltage V_{DD}, a second end of the gas-sensitive resistor is connected to the drain electrode D of the transistor p-FET, the source electrode S of the transistor p-FET is grounded, the gate electrode G is shorted to the drain electrode D, and a voltage between the drain electrode D of the transistor p-FET and the ground is used as an output voltage Vout. When alcohol is detected by the gas-sensitive sensor, the resistance of the gas-sensitive resistor of the gas-sensitive sensor is reduced, resulting in an increased current in the circuit and an increased voltage between the drain electrode D of the transistor p-FET and the ground. Because the gate electrode G of the transistor p-FET is shorted to the drain electrode D, the gate voltage and the drain voltage are increased simultaneously, causing a reduced current in the p-type transistor. The reduced current in a series circuit causes a reduced divided voltage across the gas-sensitive resistor and an increased divided voltage across the transistor p-FET, which is a self-feedback effect. With continuous self-feedback adjustment, the output voltage Vout across the transistor p-FET in the steady state is 1.5 times greater than an output voltage of a voltage dividing circuit with a fixed resistor in the related art. As shown in FIG. 3, when ozone is detected by the gas-sensitive sensor of this embodiment, the divided voltage across the transistor p-FET is reduced and the voltage between the drain electrode D of the transistor p-FET and the ground may be used as the output voltage Vout.

### Embodiment 2

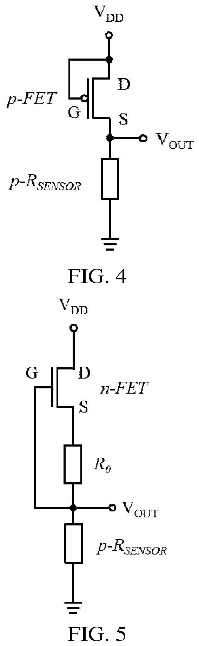

As shown in FIG. 4, V_{DD} is a test voltage, V_{OUT} is an output voltage signal, p-R_{SENSOR} is a p-type oxide semiconductor gas-sensitive resistor, p-FET is a p-type field effect transistor, and G, D and S are respectively a gate electrode, a drain electrode and a source electrode. The gas-sensitive resistor p-R_{SENSOR} of the p-type oxide semiconductor Cu₂O gas-sensitive sensor is connected in series to the p-type field effect transistor p-FET, the drain electrode D of the transistor p-FET is connected to a positive electrode of the test voltage V_{DD}, the source electrode S of the transistor p-FET is connected to a first end of gas-sensitive resistor, a second end of the gas-sensitive resistor is grounded, the gate electrode G of the transistor p-FET is shorted to the drain electrode D, and a voltage across the gas-sensitive resistor is used as an output voltage Vout. When hydrogen is detected by the gas-sensitive sensor, the resistance of the gas-sensitive resistor of the gas-sensitive sensor is increased, causing a reduced current in the circuit and a reduced voltage between the source electrode and the drain electrode of the transistor p-FET. Because the gate electrode G of the transistor p-FET is shorted to the drain electrode D, the gate voltage is reduced simultaneously as the reduction of the drain voltage, causing an increased current in the p-type transistor. The increased current in a series circuit causes an increased divided voltage across the gas-sensitive resistor, which is a self-feedback effect. With continuous self-feedback adjustment, the output voltage across the gas-sensitive resistor in the steady state is 2 times greater than an output voltage of a voltage dividing circuit with a fixed resistor in the related art. As shown in FIG. 4, when nitrogen dioxide is detected by the gas-sensitive sensor of this embodiment, the divided voltage across the gas-sensitive resistor is reduced and the voltage across the gas-sensitive resistor may be used as the output voltage Vout.

### Embodiment 3

As shown in FIG. 5, V_{DD} is a test voltage, V_{OUT} is an output voltage signal, p-R_{SENSOR} is a p-type graphene gas-sensitive resistor, n-FET is an n-type field effect transistor, G, D and S are respectively a gate electrode, a drain electrode and a source electrode, and R₀ is a series resistor. The gas-sensitive resistor p-R_{SENSOR} of the p-type oxide semiconductor LaFeO₃ gas-sensitive sensor is connected in series to the n-type field effect transistor n-FET, the drain electrode of the transistor n-FET is connected to a positive electrode of the test voltage V_{DD}, an end of the gas-sensitive resistor is grounded, the source electrode S of the transistor n-FET is connected to the gate electrode G via a fixed series resistor R₀, and a divided voltage across the gas-sensitive resistor is used as an output voltage Vout. When formaldehyde is detected by the gas-sensitive sensor, the resistance of the gas-sensitive resistor of the gas-sensitive sensor is increased, causing a reduced current in the circuit and a reduced voltage between the source electrode and the drain electrode of the transistor n-FET. Because the gate electrode of the transistor n-FET is shorted to the drain electrode via a fixed resistor, the reduced current causes an increased gate voltage, causing an increased current in the n-type field effect transistor. The increased current in a series circuit causes an increased divided voltage across the gas-sensitive resistor, which is a self-feedback effect. With continuous self-feedback adjustment, the output voltage across the gas-sensitive resistor in the steady state is 1.5 times greater than an output voltage of a voltage dividing circuit with a fixed resistor in the related art. As shown in FIG. 5, when ozone is detected by the gas-sensitive sensor of this embodiment, the divided voltage across the gas-sensitive resistor is reduced and may be used as the output voltage Vout.

### Embodiment 4

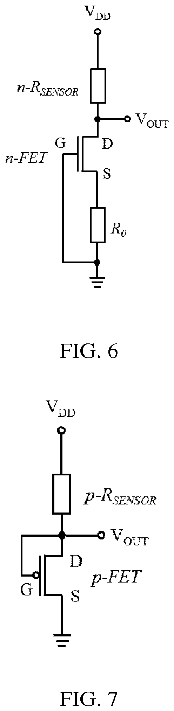

As shown in FIG. 6, V_{DD} is a test voltage, V_{OUT} is an output voltage signal, n-R_{SENSOR} is an n-type oxide semiconductor gas-sensitive resistor, n-FET is an n-type field effect transistor, G, D and S are respectively a gate electrode, a drain electrode and a source electrode, and R₀ is a series resistor. The gas-sensitive resistor of the n-type oxide semiconductor ZnO gas-sensitive sensor is connected in series to the n-type field effect transistor n-FET, an end of the gas-sensitive resistor is connected to a positive electrode of the test voltage V_{DD}, the source electrode S of the transistor n-FET is connected to the gate electrode via one fixed resistor R₀, and a divided voltage across the transistor n-FET and the fixed resistor is used as an output voltage Vout. When methane is detected by the gas-sensitive sensor, the resistance of the gas-sensitive resistor of the gas-sensitive sensor is reduced, causing an increased current in the circuit and an increased voltage between the source electrode and the drain electrode of the transistor. Because the source electrode of the transistor is connected to the gate electrode via the fixed resistor, the increased current causes a reduced gate voltage, thereby causing a reduced current in the n-type field effect transistor. The reduced current in a series circuit causes a reduced divided voltage across the gas-sensitive resistor and an increased divided voltage across the transistor n-FET and the fixed resistor, which is a self-feedback effect. With continuous self-feedback adjustment, the output voltage Vout across the transistor and the fixed resistor in the steady state is 2 times greater than an output voltage of a voltage dividing circuit with a fixed resistor in the related art. As shown in FIG. 6, when nitrogen dioxide is detected by the gas-sensitive sensor of this embodiment, the divided voltage across the transistor n-FET and the fixed resistor is reduced and may be used as the output voltage Vout.

### Embodiment 5

As shown in FIG. 7, V_{DD} is a test voltage, V_{OUT} is an output voltage signal, p-R_{SENSOR} is a p-type oxide semiconductor gas-sensitive resistor, p-FET is a p-type field effect transistor, and G, D and S are respectively a gate electrode, a drain electrode and a source electrode. The gas-sensitive resistor of the p-type graphene gas-sensitive sensor is connected in series to the p-type field effect transistor p-FET, an end of the gas-sensitive resistor is connected to a positive electrode of the test voltage V_{DD}, the source electrode S of the transistor p-FET is grounded, the gate electrode G is shorted to the drain electrode, and a voltage across the transistor p-FET is used as an output voltage Vout. When nitrogen dioxide is detected by the gas-sensitive sensor, the resistance of the gas-sensitive resistor of the gas-sensitive sensor is reduced, causing an increased current in the circuit and an increased voltage between the source electrode and the drain electrode of the transistor p-FET. Because the gate electrode of the transistor p-FET is shorted to the drain electrode, the gate voltage and the drain voltage are increased simultaneously, causing a reduced current in the p-type field effect transistor. The reduced current in a series circuit causes a reduced divided voltage across the sensor and an increased divided voltage across the transistor, which is a self-feedback effect. With continuous self-feedback adjustment, the output voltage across the transistor in the steady state is 1.5 times greater than an output voltage of a voltage dividing circuit with a fixed resistor in the related art. As shown in FIG. 7, when hydrogen is detected by the gas-sensitive sensor of this embodiment, the voltage across the transistor p-FET is reduced and may be used as the output voltage Vout.

### Embodiment 6

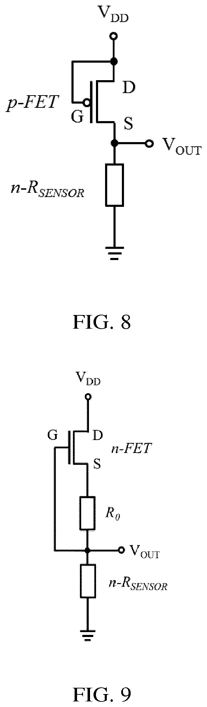

As shown in FIG. 8, V_{DD} is a test voltage, V_{OUT} is an output voltage signal, p-R_{SENSOR} is a p-type copper (II) phthalocyanine semiconductor gas-sensitive resistor, p-FET is a p-type field effect transistor, and G, D and S are respectively a gate electrode, a drain electrode and a source electrode of the p-type field effect transistor. The gas-sensitive resistor of an n-type oxide semiconductor TiO₂ gas-sensitive sensor is connected in series to the p-type field effect transistor p-FET, a first end of the gas-sensitive resistor is grounded, the gate electrode G of the transistor p-FET is shorted to the drain electrode D, the drain electrode D is connected to a positive electrode of the test voltage V_{DD}, the source electrode S is connected to a second end of the gas-sensitive resistor, and a voltage across the gas-sensitive resistor is used as an output voltage V_{OUT}. When ozone is detected by the gas-sensitive sensor, the resistance of the gas-sensitive resistor of the gas-sensitive sensor is increased, causing a reduced current in the circuit and a reduced voltage between the source electrode and the drain electrode of the transistor. Because the gate electrode of the transistor is shorted to the drain electrode, the gate voltage is reduced simultaneously as the reduction of the drain voltage, causing an increased current in the p-type transistor. The increased current in a series circuit causes an increased divided voltage across the sensor, which is a self-feedback effect. With continuous self-feedback adjustment, the output voltage across the gas-sensitive resistor in the steady state is 2 times greater than an output voltage of a voltage dividing circuit with a fixed resistor in the related art. As shown in FIG. 8, when alcohol is detected by the gas-sensitive sensor of this embodiment, the divided voltage across the gas-sensitive resistor is reduced and may be used as the output voltage Vout.

### Embodiment 7

As shown in FIG. 9, V_{DD} is a test voltage, V_{OUT} is an output voltage signal, n-R_{SENSOR} is an n-type oxide semiconductor gas-sensitive resistor, n-FET is an n-type field effect transistor, G, D and S are respectively a gate electrode, a drain electrode and a source electrode of the n-type field effect transistor, and R₀ is a series resistor. The gas-sensitive resistor of the n-type oxide semiconductor In₂O₃ gas-sensitive sensor is connected in series to the n-type field effect transistor n-FET, the drain electrode D of the transistor n-FET is connected to a positive electrode of the test voltage V_{DD}, an end of the gas-sensitive resistor is grounded, the source electrode S is connected to the source electrode S via a fixed resistor R₀, and a divided voltage across the gas-sensitive resistor is used as an output voltage Vout. When nitrogen dioxide is detected by the gas-sensitive sensor, the resistance of the gas-sensitive resistor of the gas-sensitive sensor is increased, causing a reduced current in the circuit and a reduced voltage between the source electrode and the drain electrode of the transistor. Because the source electrode of the transistor is connected to the gate electrode via the fixed resistor, the reduced current causes an increased gate voltage, causing an increased current in the n-type field effect transistor. The increased current in a series circuit causes an increased divided voltage across the gas-sensitive resistor, which is a self-feedback effect. With continuous self-feedback adjustment, the output voltage Vout across the gas-sensitive resistor in the steady state is 1.5 times greater than an output voltage of a voltage dividing circuit with a fixed resistor in the related art. As shown in FIG. 3, when methane is detected by the gas-sensitive sensor of this embodiment, the divided voltage across the gas-sensitive resistor is reduced and may be used as the output voltage Vout.

### Embodiment 8

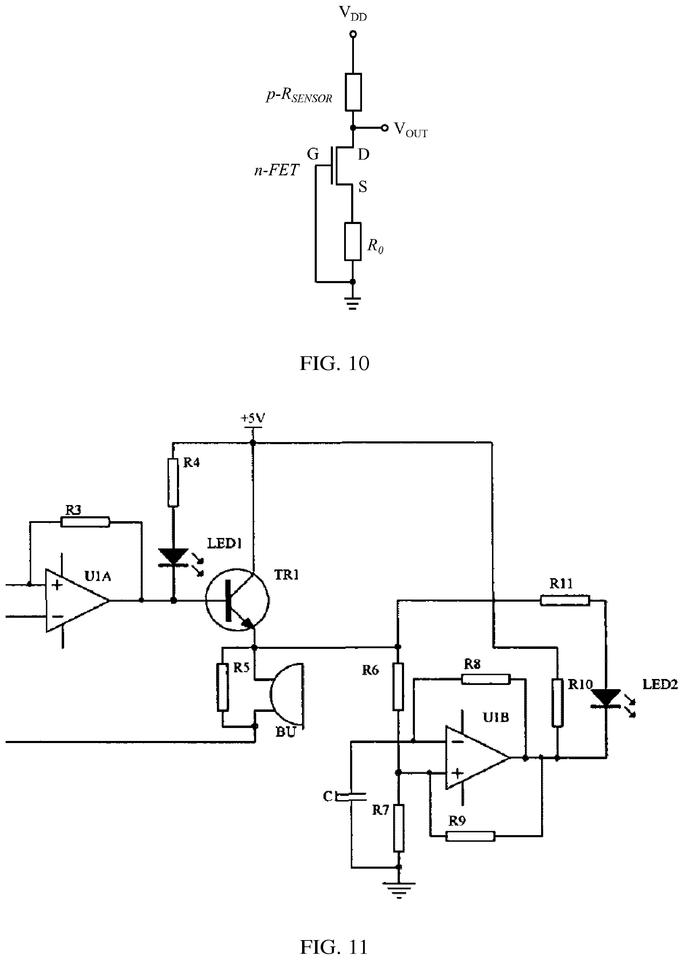

As shown in FIG. 10, V_{DD} is a test voltage, V_{OUT} is an output voltage signal, n-R_{SENSOR} is an n-type oxide semiconductor gas-sensitive resistor, n-FET is an n-type field effect transistor, G, D and S are respectively a gate electrode, a drain electrode and a source electrode of the n-type field effect transistor, and R₀ is a series resistor. The gas-sensitive resistor of a p-type organic semiconductor copper (II) phthalocyanine gas-sensitive sensor is connected in series to the n-type field effect transistor n-FET, an end of the gas-sensitive resistor is connected to a positive electrode of the test voltage V_{DD}, the source electrode S of the transistor n-FET is connected to the gate electrode G via one fixed resistor R₀, and a voltage across the transistor n-FET and the fixed resistor is used as an output voltage Vout. When ozone is detected by the gas-sensitive sensor, the resistance of the gas-sensitive resistor is reduced, causing an increased current in the circuit and an increased voltage between the source electrode and the drain electrode of the transistor n-FET. Because the source electrode of the transistor n-FET is connected to the gate electrode via the fixed resistor, the increased current causes a reduced gate voltage, thereby causing a reduced current in the n-type field effect transistor. The reduced current in a series circuit results in a reduced divided voltage across the gas-sensitive resistor and an increased divided voltage across the transistor n-FET and the fixed resistor, which is a self-feedback effect. With continuous self-feedback adjustment, the output voltage across the transistor and the fixed resistor in the steady state is 2 times greater than an output voltage of a voltage dividing circuit with a fixed resistor in the related art. As shown in FIG. 10, when formaldehyde is detected by the gas-sensitive sensor of this embodiment, the voltage across the transistor n-FET and the fixed resistor is reduced and may be used as the output voltage Vout.

Optionally, the field effect transistor in embodiments described above is an enhancement type or a depletion type.

Optionally, a gas-sensitive sensor is a sensor which operates at room temperature, or a sensor which operates at a certain temperature after being heated by a heating circuit. The room temperature may be 20°C to 25°C, and the certain temperature may be 200°C to 400°C.

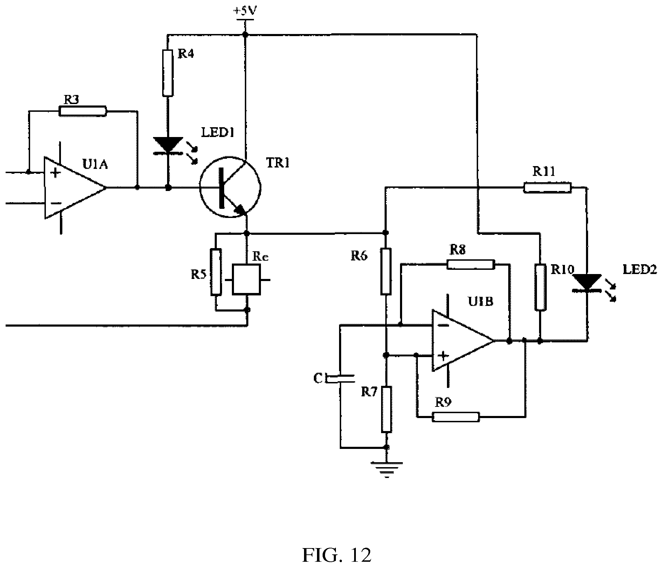

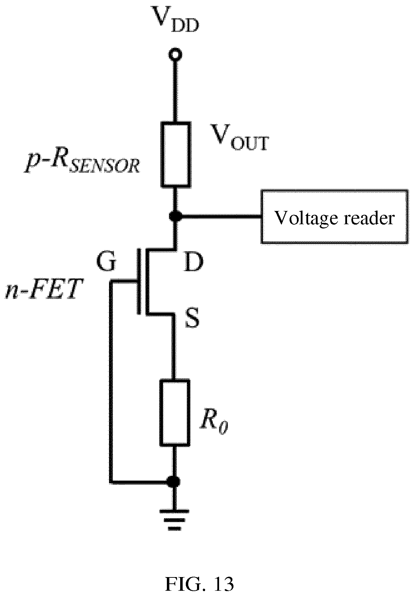

The present disclosure further provides an alarm circuit with a gas-sensitive sensor including an alarm display circuit and the self-feedback circuit described above. The alarm display circuit includes at least one of a buzzer, a light-emitting diode (LED) and a relay, an operational amplifier and a triode, where a output voltage of the self-feedback circuit is connected to the operational amplifier and the triode to drive at least one of the buzzer, the LED and the relay to give an alarm; or the alarm display circuit includes a voltage reader, where the output voltage is connected to the voltage reader, the output voltage is used as an alarm voltage, and the voltage reader is configured to digitalize the alarm voltage and output the digitalized alarm voltage. On the basis of embodiments described above, as shown in FIG. 11, an operational amplifier U1A, an operational amplifier U1B and a triode TR1 drive light-emitting diodes LED1 and LED2 and a buzzer BU. As shown in FIG. 12, an operational amplifier U1A, an operational amplifier U1B and a triode TR1 drive light-emitting diodes LED1 and LED2 and a relay Re. As shown in FIG. 13, V_{OUT} is connected to the voltage reader which digitalizes the Vout and outputs the digitalized Vout.

### INDUSTRIAL APPLICABILITY

The alarm circuit with a gas-sensitive sensor provided by the present disclosure improves alarm voltage in the steady state, the sensitivity of the alarm circuit and the alarm capability of the circuit.

## Claims

1. A self-feedback circuit, comprising a gas-sensitive sensor and a voltage dividing circuit, wherein the voltage dividing circuit comprises a field effect transistor, a gas-sensitive resistor of the gas-sensitive sensor is connected in series to a source electrode and a drain electrode of the field effect transistor, and a divided voltage across the gas-sensitive resistor or a divided voltage across the voltage dividing circuit is used as a output voltage of the self-feedback circuit.

2. The circuit according to claim 1, wherein a gate electrode of the field effect transistor is shorted to the source electrode or the drain electrode of the field effect transistor; and one of the divided voltage across the gas-sensitive resistor and a divided voltage across the field effect transistor, which is increased, is used as the output voltage.

3. The circuit according to claim 1, wherein the voltage dividing circuit comprises the field effect transistor and a series resistor, the gas-sensitive resistor of the gas-sensitive sensor is connected in series to the source electrode and the drain electrode of the field effect transistor via the series resistor; the source electrode or the drain electrode of the field effect transistor is connected in series to a gate electrode of the field effect transistor via the series resistor; and one of the divided voltage across the gas-sensitive resistor and a common divided voltage across the field effect transistor and the series resistor, which is increased, is used as the output voltage.

4. The circuit according to claim 2, wherein when the field effect transistor is a p-channel field effect transistor, the gate electrode of the p-channel field effect transistor is shorted to the drain electrode of the p-channel field effect transistor; and when the field effect transistor is an n-channel field effect transistor, the gate electrode of the n-channel field effect transistor is shorted to the source electrode of the n-channel field effect transistor.

5. The circuit according to claim 2, wherein when an n-type semiconductor gas-sensitive sensor is applied to test a reducing gas and a p-type semiconductor gas-sensitive sensor is applied to test an oxidizing gas, the divided voltage across the field effect transistor is used as the output voltage; and when the n-type semiconductor gas-sensitive sensor is applied to test the oxidizing gas and the p-type semiconductor gas-sensitive sensor is applied to test the reducing gas, the divided voltage across the gas-sensitive sensor is used as the output voltage.

6. The circuit according to claim 2 or 4, wherein the field effect transistor is an enhancement mode field effect transistor or a depletion mode field effect transistor.

7. The circuit according to claim 2 or 5, wherein the gas-sensitive sensor is a sensor which operates at room temperature, or a gas-sensitive sensor which operates at a certain temperature after being heated by a heating circuit.

8. The circuit according to claim 3, wherein when the field effect transistor is a p-channel field effect transistor, the drain electrode of the p-channel field effect transistor is connected to the gate electrode of the p-channel field effect transistor via the series resistor; and when the field effect transistor is an n-channel field effect transistor, the source electrode of the n-channel field effect transistor is connected to the gate electrode of the n-channel field effect transistor via the series resistor.

9. The circuit according to claim 3, wherein when an n-type semiconductor gas-sensitive sensor is applied to test a reducing gas and a p-type semiconductor gas-sensitive sensor is applied to test an oxidizing gas, the common divided voltage across the field effect transistor and the series resistor is used as the output voltage; and when the n-type semiconductor gas-sensitive sensor is applied to test the oxidizing gas and the p-type semiconductor gas-sensitive sensor is applied to test the reducing gas, the divided voltage across the gas-sensitive sensor is used as the output voltage.

10. The circuit according to claim 3 or 8, wherein the field effect transistor is an enhancement mode field effect transistor or a depletion mode field effect transistor.

11. The circuit according to claim 3 or 9, wherein the gas-sensitive sensor is a sensor which operates at room temperature, or a gas-sensitive sensor which operates at a certain temperature after being heated by a heating circuit.

12. The circuit according to claim 1, wherein a gate electrode of the field effect transistor is shorted to the source electrode or the drain electrode of the field effect transistor; and one of a the divided voltage across the gas-sensitive resistor and a divided voltage across the field effect transistor, which is decreased, is used as the output voltage.

13. The circuit according to claim 1, wherein the voltage dividing circuit comprises the field effect transistor and a series resistor, the gas-sensitive resistor of the gas-sensitive sensor is connected in series to the source electrode and the drain electrode of the field effect transistor via the series resistor; the source electrode or the drain electrode of the field effect transistor is connected to a gate electrode of the field effect transistor via the series resistor; and one of the divided voltage across the gas-sensitive resistor and a common divided voltage across the field effect transistor and the series resistor, which is decreased, is used as the output voltage.

14. The circuit according to claim 12, wherein when the field effect transistor is a p-channel field effect transistor, the gate electrode of the p-channel field effect transistor is shorted to the drain electrode of the p-channel field effect transistor; and when the field effect transistor is an n-channel field effect transistor, the gate electrode of the n-channel field effect transistor is shorted to the source electrode of the n-channel field effect transistor.

15. The circuit according to claim 12, wherein when an n-type semiconductor gas-sensitive sensor is applied to test a reducing gas and a p-type semiconductor gas-sensitive sensor is applied to test an oxidizing gas, the divided voltage across the gas-sensitive sensor is used as the output voltage; and when the n-type semiconductor gas-sensitive sensor is applied to test the oxidizing gas and the p-type semiconductor gas-sensitive sensor is applied to test the reducing gas, the divided voltage across the field effect transistor is used as the output voltage.

16. The circuit according to claim 13, wherein when the field effect transistor is a p-channel field effect transistor, the gate electrode of the p-channel field effect transistor is shorted to the drain electrode of the p-channel field effect transistor; and when the field effect transistor is an n-channel field effect transistor, the gate electrode of the n-channel field effect transistor is shorted to the source electrode of the n-channel field effect transistor.

17. The circuit according to claim 13, wherein when an n-type semiconductor gas-sensitive sensor is applied to test a reducing gas and a p-type semiconductor gas-sensitive sensor is applied to test an oxidizing gas, the divided voltage across the gas-sensitive sensor is used as the output voltage; and when the n-type semiconductor gas-sensitive sensor is applied to test the oxidizing gas and the p-type semiconductor gas-sensitive sensor is applied to test the reducing gas, the divided voltage across the field effect transistor is used as the output voltage.

18. An alarm circuit with a gas-sensitive sensor, comprising an alarm display circuit and the self-feedback circuit according to any one of claims 1 to 17; wherein:
the alarm display circuit comprises at least one of a buzzer, a light-emitting diode, LED, and a relay, an operational amplifier and a triode, and the output voltage of the self-feedback circuit is connected to the operational amplifier and the triode to drive the at least one of the buzzer, the LED and the relay to give an alarm; or
the alarm display circuit comprises a voltage reader, the output voltage is connected to the voltage reader, the output voltage is used as an alarm voltage, and the voltage reader is configured to digitalize the alarm voltage and output the digitalized alarm voltage.
